(19) Europäisches Patentamt
European Patent Office
Office européen des brevets

(11) EP 3 852 112 A1

(12) **EUROPEAN PATENT APPLICATION**
published in accordance with Art. 153(4) EPC

(43) Date of publication:
**21.07.2021 Bulletin 2021/29**

(21) Application number: **19858853.5**

(22) Date of filing: **13.09.2019**

(51) Int Cl.:
***G16C 20/64*** *(2019.01)* ***G16C 20/50*** *(2019.01)*

(86) International application number:
**PCT/JP2019/036073**

(87) International publication number:
**WO 2020/054840 (19.03.2020 Gazette 2020/12)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **14.09.2018 JP 2018172577**

(71) Applicant: **FUJIFILM Corporation**
**Tokyo 106-8620 (JP)**

(72) Inventor: **NAKABAYASHI, Jun**
**Ashigarakami-gun, Kanagawa 258-8538 (JP)**

(74) Representative: **Klunker IP**
**Patentanwälte PartG mbB**
**Destouchesstraße 68**
**80796 München (DE)**

# (54) COMPOUND STRUCTURE GENERATING METHOD, COMPOUND STRUCTURE GENERATING PROGRAM, AND COMPOUND STRUCTURE GENERATING DEVICE

(57) The present invention provides a method for generating a compound structure, a program for generating a compound structure, and a device for generating a compound structure, which are capable of acquiring a modified compound structure having synthetic suitability. The method for generating a compound structure includes: (A) a step of preparing a standard compound database for evaluating a synthetic aptitude, and a compound structure; (B) a step of selecting any one of an addition of an atom or an atomic group to the compound structure, or a deletion of an atom or an atomic group from the compound structure; (C) a step of, in a case of selecting the addition of an atom or an atomic group to the compound structure, bonding a new atom or a new atomic group to an atom selected from atoms included in the compound structure, or in a case of selecting the deletion an atom or an atomic group from the compound structure, deleting an selected atom or atomic group from the atoms included in the compound structure, thereby obtaining a modified compound structure; (D) a step of determining a synthetic aptitude of the modified compound structure based on information of the compound database; (E) a step of, in a case where the modified compound structure has the synthetic aptitude, probabilistically accepting the modification, or in a case where the modified compound structure does not have the synthetic aptitude, probabilistically rejecting the modification; and (F) a step of repeating the steps (B) to (E) until the compound structure which has undergone the step (E) satisfies a termination condition.

FIG. 3

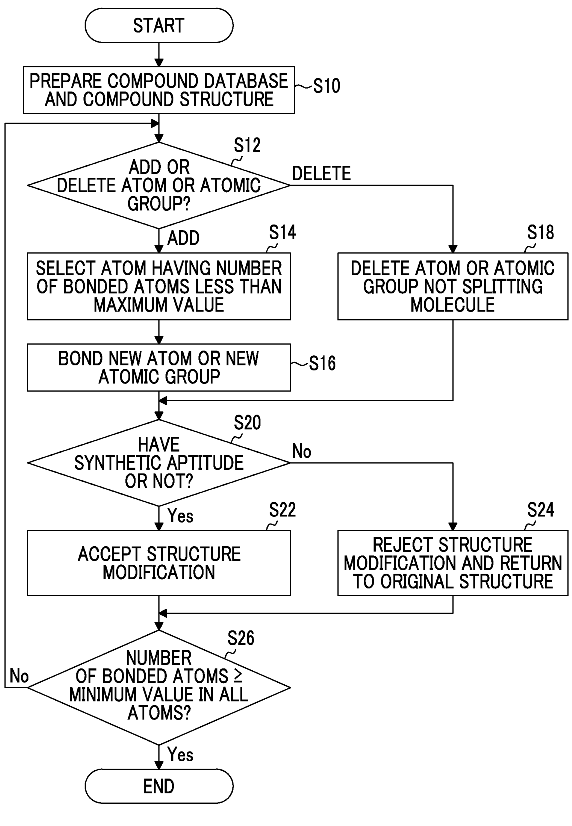


EP 3 852 112 A1

## Description

## BACKGROUND OF THE INVENTION

1. Field of the Invention

The present invention relates to a method for generating a compound structure, a program for generating a compound structure, and a device for generating a compound structure, and particularly relates to a technique for generating a compound structure having synthetic aptitude.

2. Description of the Related Art

In the related art, the search for a structure of a compound having a desired physical property value has been performed mainly by solving a "forward problem" (giving a molecular structure as a cause of the problem and obtaining a physical property value as a result). With the development of informatics in recent years, studies on a solution method of an "inverse problem" (giving a physical property value and obtaining a molecular structure having the physical property value) are rapidly progressing. For example, "Bayesian molecular design with a chemical language", Hisaki Ikebata et al., "searched on July 23, 2018", internet (https://www.ncbi.nlm.nih.gov/pubmed/28281211) is known for searching for a structure by solving the inverse problem. The "Bayesian molecular design with a chemical language", Hisaki Ikebata et al., "searched on July 23, 2018", internet (https://www.ncbi.nlm.nih.gov/pubmed/28281211) discloses that a structure having a physical property value close to the target is obtained by, giving a target of physical property value, (1) generating a plurality of initial structures (chemical structures), (2) randomly changing each structure, (3) estimating the physical property value of each structure, and (4) adopting or rejecting the change in structure based on the distance between the physical property value and the target value (in this process, the processes (2) to (4) are repeated). As described above, in order to solve the inverse problem, a technique for performing (1) to (4) is required.

In a case of performing the above-described (1) to (4), a technique capable of evaluating synthetic aptitude of the compound is required. That is, it is meaningless in a case where the chemical structures generated and/or modified on a computer are difficult to synthesize. Therefore, a technique capable of generating a compound structure having synthetic aptitude is required, and as such a technique, a technique for generating a structure by learning a partial structure or a fragment (refer to "Bayesian molecular design with a chemical language", Hisaki Ikebata et al., "searched on July 23, 2018", internet (https://www.ncbi.nlm.nih.gov/pubmed/28281211) and "RecGen (Refined Compound Generator)", Kyoto Constella Technologies Co., Ltd., "searched on July 23, 2018", internet (http://recgen.czeek.jp/recgen/)) has been known. Furthermore, a technique for updating the structure based on the evaluation results of physical property values is required (refer to "Bayesian molecular design with a chemical language", Hisaki Ikebata et al., "searched on July 23, 2018", internet (https://www.ncbi.nlm.nih.gov/pubmed/28281211)).

## SUMMARY OF THE INVENTION

In the "RecGen (Refined Compound Generator)", Kyoto Constella Technologies Co., Ltd., "searched on July 23, 2018", internet (http://recgen.czeek.jp/recgen/), in a case of connecting fragments, generation of a structure which cannot be synthesized is suppressed by preparing an overlap width portion and bonding the overlap width portion. However, in the "RecGen (Refined Compound Generator)", Kyoto Constella Technologies Co., Ltd., "searched on July 23, 2018", internet (http://recgen.czeek.jp/recgen/), the synthetic aptitude is not evaluated. In addition, the method in the "RecGen (Refined Compound Generator)", Kyoto Constella Technologies Co., Ltd., "searched on July 23, 2018", internet (http://recgen.czeek.jp/recgen/) is how to add a new structure to an existing structure, and it is difficult to delete an atom or an atomic group from the existing structure.

In order to solve the above-described inverse problem, it is required to generate a huge number of compound structures on the computer. On the other hand, in a case where a compound structure generated on the computer is difficult to synthesize, there is a problem that the structure obtained by solving the inverse problem cannot actually be synthesized.

The present invention has been studied in view of such circumstances, and an object of the present invention is to provide a method for generating a compound structure, a program for generating a compound structure, and a device for generating a compound structure, which are capable of generating a compound structure by deleting an atom or an atomic group while determining synthetic aptitude.

A method for generating a compound structure according to a first aspect includes:

(A) a step of preparing a standard compound database for evaluating a synthetic aptitude, and a compound structure;
(B) a step of selecting any one of an addition of an atom or an atomic group to the compound structure, or a deletion of an atom or an atomic group from the compound structure;

(C) a step of, in a case of selecting the addition of an atom or an atomic group to the compound structure, bonding a new atom or a new atomic group to an atom selected from atoms included in the compound structure, or in a case of selecting the deletion an atom or an atomic group from the compound structure, deleting an atom or atomic group selected from atoms or atomic groups included in the compound structure, thereby obtaining a modified compound structure;

(D) a step of determining a synthetic aptitude of the modified compound structure based on information of the compound database;

(E) a step of, in a case where the modified compound structure has the synthetic aptitude, probabilistically accepting the modification, or in a case where the modified compound structure does not have the synthetic aptitude, probabilistically rejecting the modification; and

(F) a step of repeating the steps (B) to (E) until the compound structure which has undergone the step (E) satisfies a termination condition.

According to the first aspect, a modified compound structure can be generated by adding and deleting an atom or an atomic group while determining the synthetic aptitude.

A method for generating a compound structure according to a second aspect includes that,
the compound structure prepared in the step (A) is one atom or a compound. According to the second aspect, one atom or a compound is accepted as an initial structure of the compound structure.

A method for generating a compound structure according to a third aspect includes that,
the one atom is randomly selected, or probabilistically selected based on an appearance frequency of atomic species appearing in the compound database. According to the third aspect, the degree of freedom in a case of selecting one atom is high.

A method for generating a compound structure according to a fourth aspect includes that,
in the step (B), the addition of an atom or an atomic group or the deletion of an atom or an atomic group is randomly selected, or probabilistically selected based on an appearance frequency of atomic species included in the compound database. According to the fourth aspect, in a case of the addition or deletion with respect to the compound structure, the degree of freedom in selecting the target atom or atomic group is high.

A method for generating a compound structure according to a fifth aspect includes that,
in the step (C), an atom having the number of bonded atoms less than a maximum value is probabilistically selected from the atoms included in the compound structure, and the new atom is bonded to the selected atom. According to the fifth aspect, it is easy to add a new atom or a new atomic group.

A method for generating a compound structure according to a sixth aspect includes that,
in a case of selecting the atom having the number of bonded atoms less than the maximum value in the step (C), an atom in which the number of bonded atoms does not reach a minimum value is preferentially selected, and in a case where all the atoms reach the minimum value, an atom having a large difference between the number of bonded atoms and the maximum value is preferentially or randomly selected. According to the sixth aspect, an atom which is likely to be able to bond with other atoms or atomic groups can be preferentially selected. In addition, in a case of selecting the atom having the number of bonded atoms less than the maximum value in the step (C), it is also possible to prevent one or a plurality of atoms specified in advance from being selected. According to this aspect, for example, it is possible to fix a core and modify the structure.

A method for generating a compound structure according to a seventh aspect,
in the step (C), based on the information of the compound database, the new atom is probabilistically or randomly selected from atomic species capable of bonding to the selected atom. According to the seventh aspect, since the new atom is selected from the atomic species capable of bonding to the selected atom, the degree of freedom in a case of selecting the new atom is high.

A method for generating a compound structure according to an eighth aspect includes that,
in the step (C), in a case where an atomic arrangement capable of forming a cyclic structure appears as a result of bonding the new atom to the selected atom, the cyclic structure is formed probabilistically or randomly based on the information of the compound database. According to the eighth aspect, it can be easily modified to a compound structure having a cyclic structure.

The method for generating a compound structure according to a ninth aspect includes that,
in a case of deleting an atom selected from the atoms included in the compound structure in the step (C), candidates of an atom capable of avoiding splitting the compound structure into two or more molecules are extracted, and the atom to be deleted is selected from the candidates. According to the ninth aspect, it can be modified without destroying the compound structure.

The method for generating a compound structure according to a tenth aspect includes that,
in the case of deleting an atom selected from the atoms included in the compound structure in the step (C), the atom to be deleted is selected from the candidates randomly or based on the information of the compound database. According to the tenth aspect, the degree of freedom in a case of selecting an atom to be deleted is increased. In addition, in a case of selecting an atom to be deleted, it is also possible to prevent one or a plurality of atoms specified in advance from being selected. According to this aspect, for example, it is possible to fix a core and modify the structure.

The method for generating a compound structure according to an eleventh aspect includes that,
in the synthetic aptitude of the step (D), a synthetic aptitude score of the compound structure is calculated based on an appearance frequency for each number of bonds of an atomic arrangement included in the compound database and an appearance frequency for each number of bonds of an atomic arrangement in the compound structure. According to the eleventh aspect, it is possible to suppress the generation of a compound structure not having the synthetic aptitude.

A program for generating a compound structure according to a twelfth aspect causes a computer to execute the above-described method for generating a compound structure.

A device for generating a compound structure according to a thirteenth aspect includes:

an acquisition part of acquiring a standard compound database for evaluating a synthetic aptitude, and a compound structure;
a selection part of selecting any one of an addition of an atom or an atomic group to the compound structure, or a deletion of an atom or an atomic group from the compound structure;
a modification part of, in a case of selecting the addition of an atom or an atomic group to the compound structure, bonding a new atom to an atom selected from atoms included in the compound structure, or in a case of selecting the deletion an atom or an atomic group from the compound structure, deleting an atom or atomic group selected from atoms or atomic groups included in the compound structure, thereby obtaining a modified compound structure;
a determination part of determining a synthetic aptitude of the modified compound structure based on information of the compound database;
a decision part of, in a case where the modified compound structure has the synthetic aptitude, accepting the modification, or in a case where the modified compound structure does not have the synthetic aptitude, rejecting the modification; and
a repetitive control part of repeatedly performing the processes in the selection part, the modification part, and the determination part until the compound structure which has undergone the determination part satisfies a termination condition.

According to the present invention, it is possible to generate a modified compound structure having synthetic aptitude.

## BRIEF DESCRIPTION OF THE DRAWINGS

Fig. 1 is a block diagram showing a configuration of a device for generating a compound structure.
Fig. 2 is a diagram showing a configuration of a processing part.
Fig. 3 is a flowchart showing a procedure of a method for generating a compound structure.
Fig. 4 is a diagram for explaining a portion where an atom is deleted from a compound structure.
Fig. 5 is a graph in which the vertical axis is an adoption probability p and the horizontal axis is a total score S.
Fig. 6 is a graph in which the vertical axis is the adoption probability p and the horizontal axis is the total score S.
Fig. 7 is a graph in which the vertical axis is the adoption probability p and the horizontal axis is the total score S.
Fig. 8 is a diagram showing an example of how the compound structure is modified as the process is repeated.
Fig. 9 is a diagram showing another example of how the compound structure is modified as the process is repeated.
Fig. 10 is a diagram showing still another example of how the compound structure is modified as the process is repeated.

## DESCRIPTION OF THE PREFERRED EMBODIMENTS

Hereinafter, a method for generating a compound structure, program for generating a compound structure, and device for generating a compound structure according to embodiments of the present invention will be described with reference to the accompanying drawings. In the present specification, in a case where a numerical range is expressed by using "to", the numerical range also includes numerical values of the upper limit and lower limit indicated by "to".

<Device for generating compound structure>

Fig. 1 is a block diagram showing a configuration of a device 10 for generating a compound structure (hereinafter, also simply referred to as a "generation device"). The generation device 10 is a device that generates a compound structure by, from information of a compound database, and an initial compound structure, adding an atom or the like to the compound structure or deleting an atom or the like from the compound structure to modify the compound structure; determining synthetic aptitude of the modified compound structure; deciding to adopt the change in structure; and repeating the process until a termination condition is satisfied, which can be realized using a computer. As shown in Fig. 1, the generation device 10 includes a processing part 100, a storage part 200, a display part 300, and an operation part 400, which are connected to each other to transmit and receive necessary information. Various installation forms can be adopted for these constituents, and each constituent may be installed in one place (one housing, one room, and the like), or may be installed at a distant place and connected through a network NW. In addition, the generation device 10 is connected to an external server 500 and an external compound database 510 through the network NW such as the internet, and can acquire information such as a structural formula of a compound and a crystal structure of the protein, as necessary. Fig 1 shows a case where the compound database 510 is connected through the network NW, but the compound database 510 may be prepared in the storage part 200. The compound database 510 is preferably a database with high structural diversity. However, a compound database having a specific structure (for example, coloring agent-based compounds or medicament-based compounds) can be applied depending on the purpose. It is possible to acquire a new compound structure suitable for the purpose.

<Configuration of processing part>

Fig. 2 is a diagram showing a configuration of a processing part 100. The processing part 100 includes an acquisition part 102, an add/delete selection part 104, a compound structure modification part 106, a synthetic aptitude determination part 108, a structure adoption decision part 110, a control part 112, a display control part 114, a central processing unit (CPU) 120, a read only memory (ROM) 122, and a random access memory (RAM) 124. The procedure of the method for generating a compound structure using each part of the processing part 100 will be described in detail later. The process in each part is performed under the control of the CPU 120.

The function of each part of the processing part 100 described above can be realized by using various processors. Examples of the various processors include a CPU that is a general-purpose processor which executes software (program) to realize various functions. In addition, examples of the various processors also include a graphics processing unit (GPU) which is a processor specializing in image process and a programmable logic device (PLD) which is a processor in which circuit configuration can be changed after manufacturing, such as a field programmable gate array (FPGA). Furthermore, examples of the various processors also include a dedicated electric circuit which is a processor having a circuit configuration specifically designed to execute a specific process, such as an application specific integrated circuit (ASIC).

The functions of each part may be realized by one processor, or may be realized by a plurality of processors of the same type or different types (for example, a plurality of FPGAs, a combination of CPU and FPGA, or a combination of CPU and GPU). In addition, a plurality of functions may be realized by one processor. As an example of configuring a plurality of functions with one processor, firstly, an aspect that, as typified by a computer such as a client and a server, one processor is configured by a combination of one or more CPUs and software, and this processor realizes the plurality of functions is exemplified. Secondly, an aspect that, as typified by a system on chip (SoC), uses a processor which realizes the functions of the entire system with a single integrated circuit (IC) chip is exemplified. As described above, various functions are composed by using one or more of the above-described various processors as a hardware structure. Furthermore, the hardware structure of these various processors is more specifically an electric circuit (circuitry) in which circuit elements such as semiconductor elements are combined. This electric circuit may be an electric circuit which realizes the above-described functions by using logical sum, logical product, logical negation, exclusive logical sum, and logical operation of a combination thereof.

In a case where the above-described processor or electric circuit executes a software (program), a processor-readable code (computer-readable code) of the software to be executed is stored in a non-temporary recording medium such as ROM 122 (refer to Fig. 2), and the processor refers to the software. The software stored in the non-temporary recording medium includes the program (program for generating a compound structure) for executing the method for generating a compound structure according to the embodiment of the present invention. The code may be recorded in a non-temporary recording medium such as various magneto-optical recording devices and semiconductor memories instead of ROM 122. In a case of processing using a software, for example, RAM 124 is used as a temporary storage area, and for example, data stored in an electronically erasable and programmable read only memory (EEPROM) (not shown) can be referred to.

<Configuration of storage part>

The storage part 200 is configured of a non-temporary recording medium such as a digital versatile disk (DVD), a hard disk, and various semiconductor memories, and a control part thereof, and can store compound structures (initial compound structure and modified compound structure); a compound database; atomic species obtained based on the compound database, an atomic arrangement, and an appearance frequency of each result; a synthetic aptitude score; and the like.

Configuration of display part and operation part>

The display part 300 includes a monitor 310 (display device), and can display the input image, the information stored in the storage part 200, the result of process by the processing part 100, and the like. The operation part 400 includes a keyboard 410 and a mouse 420 as input devices and/or pointing devices, and the user can perform operations necessary for executing the method for generating a compound structure through these devices and a screen of the monitor 310. For example, the user can perform designation of process start instruction, input of an initial compound structure, hyperparameter for controlling the difficulty of synthetic aptitude, and the like.

<Procedure of method for generating compound structure>

Fig. 3 is a flowchart showing a procedure of a method for generating a compound.

<Preparation of compound database and compound structure>

A compound database for evaluating synthetic aptitude and a compound structure (initial structure) are prepared (Step S10). Data stored in the storage part 200 may be used as these data, or these data may be acquired from the external server 500 and the compound database 510 through the network NW. A compound database 510 including compounds suitable for the purpose is selected. What kind of data may be prepared may be decided according to the user's instruction input through the operation part 400.

The compound structure (initial structure) can be selected from the compound database 510, or may be input by the user through the operation part 400. In a case of selecting the compound structure from the compound database 510, the compound structure can be selected randomly from the compound database 510, or can be selected probabilistically based on the appearance frequency in the compound database 510. Random selection means that the selection is performed randomly, and the probabilistic selection means that the selection is performed based on some weighting.

An example of a case of selecting in one atom unit based on the appearance frequency in the compound database 510 will be described. Table 1 is a table in which atomic species of the compound database 510 are arranged in descending order of the appearance frequency. The atomic species include an atom, which is included in each compound included in the compound database 510, and an electronic state thereof (type of bonding). As shown in Table 1, the appearance frequency of "C.ar" is the highest, the appearance frequency of "C.3" is the second highest, and the appearance frequency of "Lr" is the lowest.

In Table 1, ar means aromatic, and "C.ar" means an aromatic carbon. The minimum value of the number of bonded atoms of "C.ar" is 2, and the maximum value thereof is 3. "C.3" is a carbon of $sp^3$ hybrid orbital, and the minimum value of the number of bonded atoms is 1 and the maximum value of the number of bonded atoms is 4. For example, as an atom name including the electronic state of the atom, the mol2 format of Tripos can be applied. "C.1" is a carbon of $sp^1$ hybrid orbital, and "C.2" is a carbon of $sp^2$ hybrid orbital.

In a case of selecting the initial structure probabilistically, the atomic species are weighted according to the appearance frequency. The initial structure is selected according to the weighting. For example, atomic species with a high appearance frequency are selected. On the other hand, in a case of random, the initial structure is selected randomly from all atomic species. For example, atomic species with a low appearance frequency may be selected.

[Table 1]

| Atomic species | Appearance frequency |
|---|---|
| C.ar | 868432654 |
| C.3 | 693030631 |
| ⋮ | ⋮ |
| Lr | 1 |

The data of the compound database and the compound structure (initial structure) are input to the processing part 100 through the acquisition part 102. The compound structure (initial structure) can be accepted to be either one atom or a compound.

As the compound database, PubChem (http://pubchem.ncbi.nlm.nih.gov/search/), DrugBank (http://www.drugbank.ca/), and the like can be used.

<Addition or deletion of atom or atomic group to compound structure>

The add/delete selection part 104 determines and selects to add an atom or an atomic group to the compound structure, or to delete an atom or an atomic group from the compound structure (Step S12). Here, immediately after the start of the method for generating a compound structure, the compound structure in the step S12 means a compound structure as the initial structure. On the other hand, after passing through the step S26 described later, the compound structure in the step S12 is a modified compound structure. In the step S12, a case of addition or deletion in one atom unit with respect to the compound structure and a case of addition or deletion in an atomic group (group of two or more atoms) unit with respect to the compound structure are accepted.

In the step S12, in a case where the compound structure prepared in the step S10 is one atom, the addition of an atom or an atomic group to the compound structure is selected.

In the step S12, by setting a threshold value for a molecular weight of the compound structure and increasing a probability of selecting the deletion of an atom or an atomic group in a case where the molecular weight of the compound structure exceeds the threshold value, the molecular weight of the generated compound structure can be limited.

In the step S12, the addition of an atom or an atomic group or the deletion of an atom or an atomic group can be randomly selected, or probabilistically selected based on the appearance frequency of the atomic species included in the compound database.

<Acquisition of modified compound structure>

In a case of selecting an addition of an atom or an atomic group to the compound structure in the step S12, the compound structure modification part 106 selects an atom having the number of bonded atoms less than the maximum value from atoms included in the compound structure (Step S14), and then bonds a new atom or a new atomic group to the atom selected from atoms included in the compound structure (Step S16). In addition, in a case of selecting a deletion of an atom or an atomic group from the compound structure in the step S12, the compound structure modification part 106 deletes the atom or atomic group selected from the atoms or atomic groups included in the compound structure (Step S18).

In the step S14, the compound structure modification part 106 examines the number of bonded atoms of each atom of the compound structure. The number of bonded atoms of each atom can be obtained from Table 1 filled in based on the compound database 510. For example, by selecting one atom from the compound structure and searching for the selected one atom from Table 1, the number of bonded atoms of the selected one atom can be obtained. The number of bonded atoms can be obtained in the same way for all the atoms included in the compound structure. All the atoms for which the number of bonded atoms has been obtained are listed, and from the list, one atom is probabilistically selected as an atom or an atom to which an atomic group is added.

A hydrogen atom included in the compound structure can be omitted unless it is necessary to consider the hydrogen atom. This is because the compound structure is complicated in a case where the hydrogen atom is extracted. In the compound structure, in a case of selecting one atom having the number of bonded atoms less than the maximum value as the atom to which an atom or an atomic group is added, it is preferable that an atom in which the number of bonded atoms does not reach the minimum value is preferentially selected. In a case where all the atoms in the compound structure reach the minimum value, it is preferable that a probability in which an atom having a large difference between the number of bonded atoms and the maximum value is selected increases.

In the step S16, based on the compound database 510, the compound structure modification part 106 probabilistically selects one new atom or new atomic group, which can be bonded to the atom selected in the step S14, from an atomic arrangement (atomic species and type of bonding (single bond, double bond, and the like)), and forms a bonding.

Table 2 is a table of atomic arrangements, which is filled in based on the compound database 510. Table 2 shows atomic arrangements (atomic species, type of bonding, and appearance frequency) which can be bonded to "C.3", in a case where the atom selected in the step S14 is "C.3". In Table 2, "-" represents a single bond, "=" represents a double bond, "#" represents a triple bond, and ":" represents an aromatic bond.

[Table 2]

| Bonding pattern including C.3 | |
|---|---|
| Atomic arrangement | Appearance frequency |
| C.3 - C.3 | 427869724 |
| C.3 - C.ar | 106701209 |
| ⋮ | ⋮ |
| C.3 - Kr | 1 |

For example, in a case of probabilistically selecting a new atom bonded to the atom selected in the step S14, it is weighted according to the appearance frequency of the atomic arrangement. The atomic arrangement is selected according to the weighting, the atom included in the atomic arrangement is bonded to the atom selected in the step S14 as the new atom. On the other hand, in a case of random, it is selected randomly from all atomic arrangements.

In a case where an atomic arrangement capable of forming a cyclic structure appears as a result of bonding the new atom, the cyclic structure can be probabilistically formed. As for the probability of forming a cyclic structure, it is preferable to directly estimate a ratio in a case where the atomic arrangement is a cyclic structure in the compound database 510. However, a cyclic structure can be randomly formed.

In the step S18, the compound structure modification part 106 determines whether or not the compound structure is split into two or more molecules in a case where an atom in the compound structure is deleted. For example, in a compound structure shown in Fig. 4, atoms indicated by an arrow A are extracted as a candidate capable of avoiding splitting the compound structure into two or more molecules. On the other hand, atoms indicated by an arrow B is not extracted as the candidate. This is because, in a case where the atom indicated by the arrow B is deleted, the compound structure is split into two or more molecules.

As for the atom to be deleted from the compound structure, for example, candidate atoms are listed. The atom to be deleted can be randomly selected from the list. In addition, as the atom to be deleted, the same atom as an atom with a low appearance frequency in the compound database 510 can also be preferentially selected from the list.

The compound structure modification part 106 acquires a modified compound structure by passing through the step S16 or the step S18.

<Determination of synthetic aptitude>

The synthetic aptitude determination part 108 determines a synthetic aptitude of the modified compound structure, which is acquired by the compound structure modification part 106, based on information of the compound database 510 (Step S20).

The determination of the synthetic aptitude is performed, for example, by the following procedure. The procedure includes (1) extracting an atomic arrangement from a compound stored in a compound database and obtaining an appearance frequency of the atomic arrangement, (2) extracting an atomic arrangement from a modified compound structure and obtaining an appearance frequency of the atomic arrangement, (3) calculating, for each atomic arrangement in the compound structure, a frequency with which the atomic arrangement in the modified compound structure appears in the compound obtained from the compound database, as a partial score, based on the number of bonds included in the atomic arrangement in the modified compound structure and an appearance frequency of an atomic arrangement which corresponds to the atomic arrangement and is obtained from the compound database, using a function in which a numerical value decreases as the number of bonds and appearance frequency in the atomic arrangement in the modified compound structure increase, and (4) evaluating a synthetic aptitude by summing the calculated partial scores and obtaining a total score which is a synthetic aptitude score of the compound structure.

Tables 3 and 4 are tables of atomic arrangements, which are filled in based on the compound database 510, in which the number of bonds is used as the standard. Tables 3 and 4 include the number of bonds and the atomic arrangement (atomic species, type of bonding, and appearance frequency).

[Table 3]

| One bond | |
|---|---|
| Atomic arrangement | Appearance frequency |
| C.ar : C.ar | 799082034 |
| C.3 - C.3 | 427869724 |

(continued)

| One bond | |
|---|---|
| Atomic arrangement | Appearance frequency |
| ⋮ | ⋮ |
| I # S.3 | 1 |

In Table 3, "S.3" is a sulfur of $sp^3$ hybrid orbital.

[Table 4]

| Five bonds | |
|---|---|
| Atomic arrangement | Frequency of appearance |
| C.ar : C.ar : C.ar : C.ar : C.ar : C.ar | 765522244 |
| C.3 - C.3 - C.3 - C.3 - C.3 - C.3 | 180664124 |
| ⋮ | ⋮ |
| C.ar - C.ar : C.ar = C.2 - N.p13 - O.co2 | 1 |

In Table 4, "N.pl3" is a nitrogen trigonal planer, and "O.co2" is an oxygen in carboxylate and phosphate groups.

Atomic arrangements are extracted from the modified compound structure for each of the number of bonds. The appearance frequency of the extracted atomic arrangement in the modified compound structure is obtained. Table 5 is a table of atomic arrangements, which is obtained from a modified compound structure.

[Table 5]

| One bond | |
|---|---|
| Atomic arrangement | Appearance frequency |
| C.ar : C.ar | 6 |
| 0.3 - C.ar | 1 |
| Br - 0.3 | 1 |
| Br - (edge) | 1 |

In Table 5, "O.3" is an oxygen of sp hybrid orbital, and edge means that the terminal of the molecule.

In a case where n(substr) represents the number of bonds of the atomic arrangement, f(substr) represents the appearance frequency of the atomic arrangement in the compound database, and f1 (substr) represents the appearance frequency of the atomic arrangement in the modified compound structure, the partial score s(substr) can be obtained by Expression (1).

$$s(\text{substr}) = \frac{f1(\text{substr})}{n(\text{substr}) \times (f(\text{substr}) + 1)} \quad (1)$$

For example, a partial score of "C.ar : C.ar" included in the modified compound structure can be calculated as follows. From Table 3, the appearance frequency of "C.ar : C.ar" in the compound database 510 is 799082034. From Table 5, the appearance frequency of "C.ar : C.ar" in the modified compound structure is 6.

$$s(\text{C.ar : C.ar}) = \text{f1}(\text{C.ar : C.ar}) / (\text{n}(\text{C.ar : C.ar}) \times (\text{f}(\text{C.ar : C.ar}) + 1)) = 6 / (1 \times (799082034 + 1)) = 7.5 \times 10^{-9}$$

A total score S can be obtained by obtaining partial scores for all the atomic arrangements included in the modified compound structure and summing the partial scores s.

The determination of the synthetic aptitude can be performed by setting a threshold value for the total score S. In a case where the total score S is equal to or less than the set threshold value, the modified compound structure is determined to have the synthetic aptitude.

In a case where a threshold value is set for the total score S, a compound structure having a total score S more than the threshold value is not generated at all. In fact, in a case of performing, with respect to the compound structure (including the initial structure and the modified compound structure), the process of the addition of a new atom or a new atomic group and the deletion of an atom or an atomic group, a compound structure having a total score S less than the threshold value may be acquired after passing through the compound structure having a total score S more than the threshold value. Therefore, it is necessary to determine a synthetic aptitude which can accept the compound structure having the total score S more than the threshold value.

In a case where an adoption probability is represented by p, the total score is represented by S, and the hyperparameter is represented by $\sigma$, the determination of the synthetic aptitude which can accept the compound structure having the total score S more than the threshold value can be probabilistically performed by Expression (2). Adjustment of the synthesis difficulty of the modified compound structure is performed by changing the value of the hyperparameter $\sigma$.

$$p = \exp[-S/\sigma] \qquad (2)$$

Next, the adjustment of the synthesis difficulty will be described. Figs. 5 to 7 are graphs in which the vertical axis is the adoption probability p and the horizontal axis is the total score S. In Figs. 5 and 6, the result in a case where the hyperparameter $\sigma$ is 0.1 is plotted with a solid line, and the result in a case where the hyperparameter $\sigma$ is 10 is plotted with a broken line.

As shown in the graph of Fig. 5, in a case where the hyperparameter $\sigma$ is 0.1, the adoption probability p is almost 0% in a case where the total score S is around 0.5. On the other hand, in a case where the hyperparameter $\sigma$ is 10, the adoption probability p is 90% or more in a case where the total score S is around 0.5. That is, in a case where the hyperparameter $\sigma$ is 10, a compound structure having a large total score S (so-called structure having low synthetic aptitude) is determined to have the synthetic aptitude, and the compound structure is accepted.

As shown in the graph of Fig. 6, in a case where the hyperparameter $\sigma$ is 10, the adoption probability p is almost 0% in a case where the total score S is around 50. From Figs. 5 and 6, it can be understood that the value of the hyperparameter $\sigma$ allows the adjustment of the difficulty of the synthetic aptitude.

In a case where the hyperparameter $\sigma$ is $\infty$, the adoption probability p is 100% regardless of the total score S. Expression (2) includes a case where the synthetic aptitude is determined without setting a threshold value for the total score S.

In addition, in a case where the adoption probability is represented by p, the total score is represented by S, the hyperparameter is represented by $\sigma$, and a parameter is represented by d, the determination of the synthetic aptitude can be probabilistically performed by Expression (3) which is an extended exponential function.

$$p = \exp\left[-\left(\frac{S}{\sigma}\right)^{d}\right] \qquad (3)$$

In Fig. 7, the result in a case where the parameter d is 1 is plotted with a solid line, the result in a case where the parameter d is 2 is plotted with a broken line, and the result in a case where the parameter d is 10000 is plotted with a dotted line.

In a case where the parameter d is 1, Expression (3) is the same as the function of the adoption probability p represented by Expression (2). In a case where the parameter d is increased, Expression (3) changes as follows.

In a case where the parameter d is $\infty$, the adoption probability p is 1/e in a case where total score S = hyperparameter $\sigma$. In addition, the adoption probability p is 1 in a case where total score S < hyperparameter $\sigma$, and the adoption probability p is 0 in a case where total score S > hyperparameter $\sigma$. The plotted graphs asymptotically approach the so-called Heaviside step function. In Fig. 7, in a case where the parameter d is 10000, the dotted graph is closer to the Heaviside step function. This means that the expanded exponential function (Expression (3)) includes a case where a threshold value is set for the total score S itself in the limit in which d is $\infty$.

As long as the synthetic aptitude can be determined, the determination of the synthetic aptitude is not limited to the above-described detail.

<Acceptance and rejection of compound structure>

The structure adoption decision part 110 probabilistically accepts the modification in a case where the modified compound structure has the synthetic aptitude (Step S22), or probabilistically rejects the modification in a case where the modified compound structure does not have the synthetic aptitude (Step S24).

Here, the "probabilistically" can be realized by applying the adoption probability p in the step S20.

In the method for generating a compound structure according to the embodiment, the synthetic aptitude is determined every time the compound structure is modified. By the determination of the synthetic aptitude, it is possible to suppress the generation of a compound structure which is difficult to synthesize. On the other hand, by adjusting the adoption probability p of the synthetic aptitude, a compound structure having low synthetic aptitude can be accepted, which increases the degree of freedom in modifying the compound structure and promotes the generation of new compound structure.

<Repetition of process>

In a case where, as described above, the structure adoption decision part 110 probabilistically accepts the modification of the compound structure in a case where the modified compound structure has the synthetic aptitude (Step S22), or probabilistically rejects the modification of the compound structure in a case where the modified compound structure does not have the synthetic aptitude (Step S24), the control part 112 determines whether or not a termination condition is satisfied (Step S26). For example, the control part 112 can determine that "the termination condition is satisfied" in a case where all the atoms included in the compound structure are equal to or more than the minimum value with respect to the number of bonded atoms. In a case where the termination condition is not satisfied, the control part 112 repeatedly performs the steps S12 to S26. On the other hand, in a case where the control part 112 determines that "the termination condition is satisfied", the generation of the compound structure is terminated.

<Example>

The present invention will be specifically described with reference to the example. Even in this example, the process can be performed by the generation device 10 shown in Figs. 1 and 2 and the flowchart (method for generating a compound structure and process of the program for generating a compound structure) shown in Fig. 3.

As shown in Fig. 8, in the preparation step (Step S10), PubChem available on the internet is prepared as the compound database 510. As a prepared compound structure as the initial structure, "C.3" is probabilistically selected from the appearance frequency of atomic species appearing in the compound database 510 (refer to Table 1).

In the step of adding or deleting an atom or an atomic group (Step S12), the addition or deletion of an atom or an atomic group is selected probabilistically. However, since "C.3" is one atom, the deletion of an atom or an atomic group is not selected, but the addition of an atom or an atomic group is selected.

In the step of selecting an atom having the number of bonded atoms less than the maximum value (Step S14), "C.3" having the number of bonded atoms less than the maximum value is selected as an atom to which a new atom or a new atomic group is bonded. Here, the minimum value of the number of bonded atoms of "C.3" is 1 and the maximum value thereof is 4. Since "C.3" is one atom, "C.3" is in a state in which the number of bonded atoms does not reach the minimum value.

In the step of bonding a new atom or atomic group (Step S16), the atomic arrangement capable of bonding to "C.3" and the type of bonding are selected probabilistically from the list (refer to Table 2) filled in based on the compound database 510. In the example, "C.3 - C.ar" which has the second highest appearance frequency is selected. In Fig. 8, in order to facilitate understanding, a bond of the compound structure before the synthetic aptitude is determined (before accepting the modification in structure) is indicated by a thin line, and a bond of the compound structure after the synthetic aptitude is determined (after accepting the modification in structure) is indicated by a thick line.

Next, in the step of determining the synthetic aptitude (Step S20), the synthetic aptitude of "C.3 - C.ar" is determined. In the example, Expression (2) in which a total score S and a hyperparameter $\sigma = 0.1$ are included is applied to the calculation of an adoption probability p of the synthetic aptitude. The adoption probability p of this modification in structure is calculated by Expression (4).

$$S = 9.37 \times 10^{-9}$$
$$p = \exp\left[-\frac{9.37 \times 10^{-9}}{0.1}\right] \cong 1 \qquad (4)$$

Since the adoption probability p is almost 1, "C.3 - C.ar" is determined to have the synthetic aptitude. The change in structure is accepted almost 100% (Step S22). As shown in accepting the modification in structure of Fig. 8, the bond of "C.3 - C.ar" changes from the thin line to the thick line.

As a result of adding (bonding) new atom, "C.3" reaches 1 which is the minimum value of the number of bonded atoms. On the other hand, "C.ar" does not reach 2 which is the minimum value of the number of bonded atoms. It is determined that the termination condition is not satisfied (Step S26). The process returns to the step S12, and is repeated. After that, the same process is repeated 5 times.

At the sixth time, as shown in Fig. 8, a modified compound structure is acquired as a result of newly bonding "C.ar - C.ar" indicated by an arrow 1 to the compound structure. Based on the compound database 510, "C.ar : C.ar : C.ar : C.ar : C.ar : C.ar" included in the modified compound structure can form a cyclic structure with a probability of 88%. Assuming that the cyclic structure is adopted as a result of evaluation with random numbers, a cyclic structure further bonded with C.ar - C.ar" indicated by an arrow 2 is acquired as a modified compound structure. This modified compound structure is determined for the synthetic aptitude.

The adoption probability p of this modification in structure is calculated by Expression (5).

$$S = 1.00 \times 10^{-7}$$
$$p = \exp\left[-\frac{1.00 \times 10^{-7}}{0.1}\right] \cong 1 \qquad (5)$$

Since the adoption probability p is almost 1, "C.ar - C.ar" is determined to have the synthetic aptitude (Step S20). The change in structure is accepted almost 100% (Step S22). As shown in accepting the modification in structure of Fig. 8, the bond of C.ar : C.ar" changes from the thin line to the thick line.

Regarding a possible range of the number of bonded atoms, "C.3" is 1 to 4 and "C.ar:" is 2 and 3. The atoms included in the modified compound structure satisfy the possible ranges of the number of bonded atoms, which is the termination condition (Step S26). The modification of the compound structure is completed, and the method for generating a compound structure is terminated.

In Fig. 8, one atom is prepared as the initial structure. The initial structure is not limited to one atom. The initial structure may be a compound structure generated by the method for generating a compound structure. In Fig. 9, an initial structure is a compound structure generated by the method for generating a compound structure. It is confirmed that the modified compound structure can be acquired by repeating the process 11 times from the initial structure.

By the method for generating a compound structure according to the embodiment, as shown in Fig. 10, it is possible to confirm an actual compound structure called methyl yellow.

The embodiments and examples of the present invention have been described above, but the present invention is not limited to the above-described aspects, and various modifications are possible without departing from the gist of the present invention.

Explanation of References

10: device for generating compound structure
100: processing part
102: acquisition part
104: add/delete selection part
106: compound structure modification part

108: synthetic aptitude determination part
110: structure adoption decision part
112: control part
114: display control part
120: CPU
200: storage part
300: display part
310: monitor
400: operation part
410: keyboard
420: mouse
500: external server
510: compound database
NW: network

**Claims**

1. A method for generating a compound structure, the method comprising:

    (A) a step of preparing a standard compound database for evaluating a synthetic aptitude, and a compound structure;
    (B) a step of selecting any one of an addition of an atom or an atomic group to the compound structure, or a deletion of an atom or an atomic group from the compound structure;
    (C) a step of, in a case of selecting the addition of an atom or an atomic group to the compound structure, bonding a new atom or a new atomic group to an atom selected from atoms included in the compound structure, or in a case of selecting the deletion of an atom or an atomic group from the compound structure, deleting an atom or atomic group selected from atoms or atomic groups included in the compound structure, thereby obtaining a modified compound structure;
    (D) a step of determining a synthetic aptitude of the modified compound structure based on information of the compound database;
    (E) a step of, in a case where the modified compound structure has the synthetic aptitude, probabilistically accepting the modification, or in a case where the modified compound structure does not have the synthetic aptitude, probabilistically rejecting the modification; and
    (F) a step of repeating the steps (B) to (E) until the compound structure which has undergone the step (E) satisfies a termination condition.

2. The method for generating a compound structure according to claim 1,
wherein the compound structure prepared in the step (A) is one atom or a compound.

3. The method for generating a compound structure according to claim 2,
wherein the one atom is randomly selected, or probabilistically selected based on an appearance frequency of atomic species appearing in the compound database.

4. The method for generating a compound structure according to any one of claims 1 to 3,
wherein, in the step (B), the addition of an atom or an atomic group or the deletion of an atom or an atomic group is randomly selected, or probabilistically selected based on an appearance frequency of atomic species included in the compound database.

5. The method for generating a compound structure according to any one of claims 1 to 4,
wherein, in the step (C). an atom having the number of bonded atoms less than a maximum value is probabilistically selected from the atoms included in the compound structure, and the new atom is bonded to the selected atom.

6. The method for generating a compound structure according to claim 5,
wherein, in a case of selecting the atom having the number of bonded atoms less than the maximum value in the step (C), an atom in which the number of bonded atoms does not reach a minimum value is preferentially selected, and in a case where all the atoms reach the minimum value, an atom having a large difference between the number of bonded atoms and the maximum value is preferentially or randomly selected.

**7.** The method for generating a compound structure according to any one of claims 1 to 6, wherein, in the step (C), based on the information of the compound database, the new atom is probabilistically or randomly selected from atomic species capable of bonding to the selected atom.

**8.** The method for generating a compound structure according to claim 7, wherein, in the step (C), in a case where an atomic arrangement capable of forming a cyclic structure appears as a result of bonding the new atom to the selected atom, the cyclic structure is formed probabilistically or randomly based on the information of the compound database.

**9.** The method for generating a compound structure according to any one of claims 1 to 8, wherein, in a case of deleting an atom selected from the atoms included in the compound structure in the step (C), candidates of an atom capable of avoiding splitting the compound structure into two or more molecules are extracted, and the atom to be deleted is selected from the candidates.

**10.** The method for generating a compound structure according to claim 9, wherein, in the case of deleting an atom selected from the atoms included in the compound structure in the step (C), the atom to be deleted is selected from the candidates randomly or based on the information of the compound database.

**11.** The method for generating a compound structure according to any one of claims 1 to 10, wherein, in the synthetic aptitude of the step (D), a synthetic aptitude score of the compound structure is calculated based on an appearance frequency for each number of bonds of an atomic arrangement included in the compound database and an appearance frequency for each number of bonds of an atomic arrangement in the compound structure.

**12.** A program for generating a compound structure, which causes a computer to execute the method for generating a compound structure according to any one of claims 1 to 11.

**13.** A non-temporary and computer-readable recording medium, which causes a computer to execute the program according to claim 12 in a case where a command stored in the recording medium is read by the computer.

**14.** A device for generating a compound structure, the device comprising:

an acquisition part of acquiring a standard compound database for evaluating a synthetic aptitude, and a compound structure;
a selection part of selecting any one of an addition of an atom or an atomic group to the compound structure, or a deletion of an atom or an atomic group from the compound structure;
a modification part of, in a case of selecting the addition of an atom or an atomic group to the compound structure, bonding a new atom to an atom selected from atoms included in the compound structure, or in a case of selecting the deletion of an atom or an atomic group from the compound structure, deleting an atom or atomic group selected from atoms or atomic groups included in the compound structure, thereby obtaining a modified compound structure;
a determination part of determining a synthetic aptitude of the modified compound structure based on information of the compound database;
a decision part of, in a case where the modified compound structure has the synthetic aptitude, accepting the modification, or in a case where the modified compound structure does not have the synthetic aptitude, rejecting the modification; and
a repetitive control part of repeatedly performing the processes in the selection part, the modification part, and the determination part until the compound structure which has undergone the determination part satisfies a termination condition.

FIG. 1

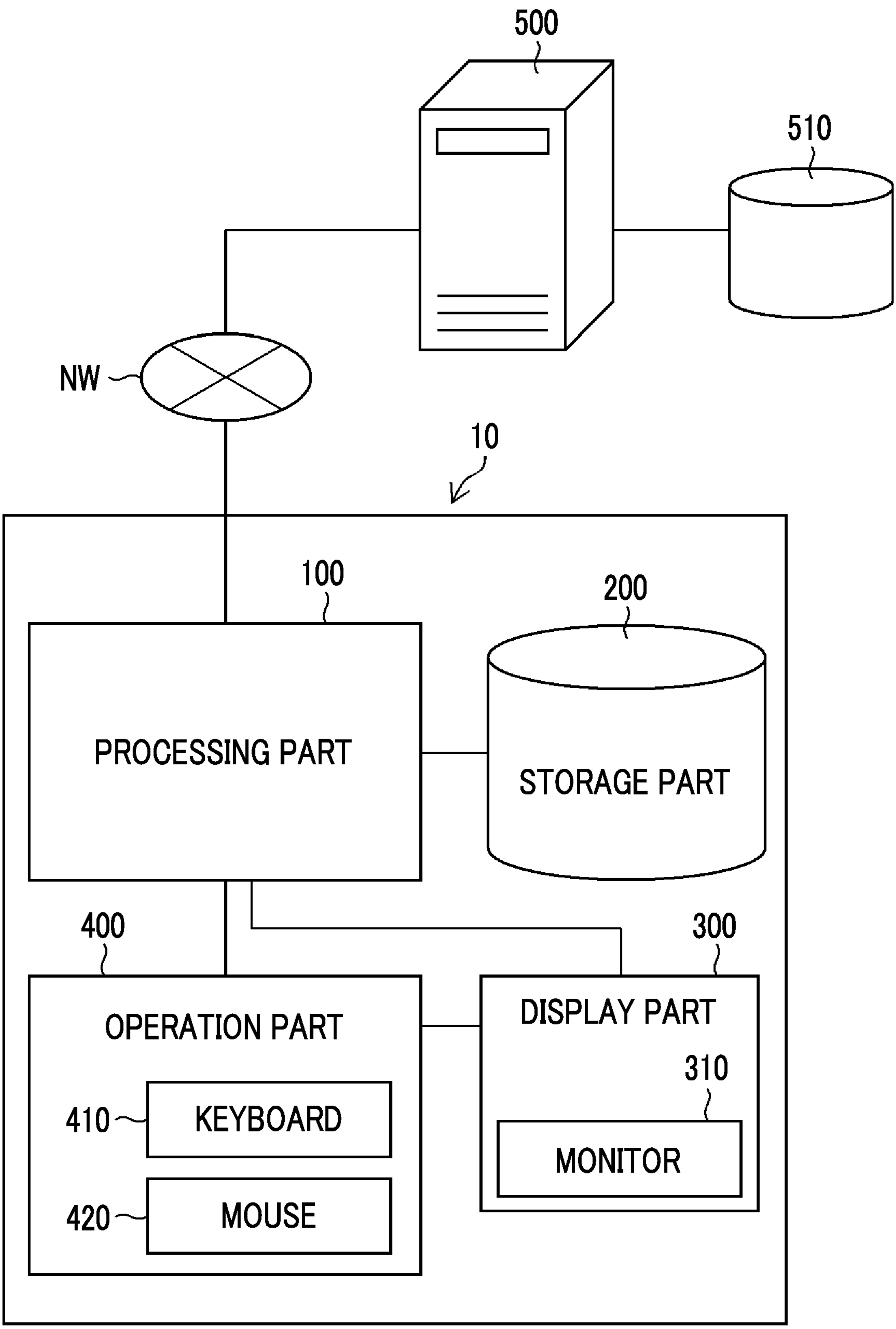
500
510
NW
10
100
PROCESSING PART
200
STORAGE PART
400
300
OPERATION PART
DISPLAY PART
310
410
KEYBOARD
MONITOR
420
MOUSE

FIG. 2

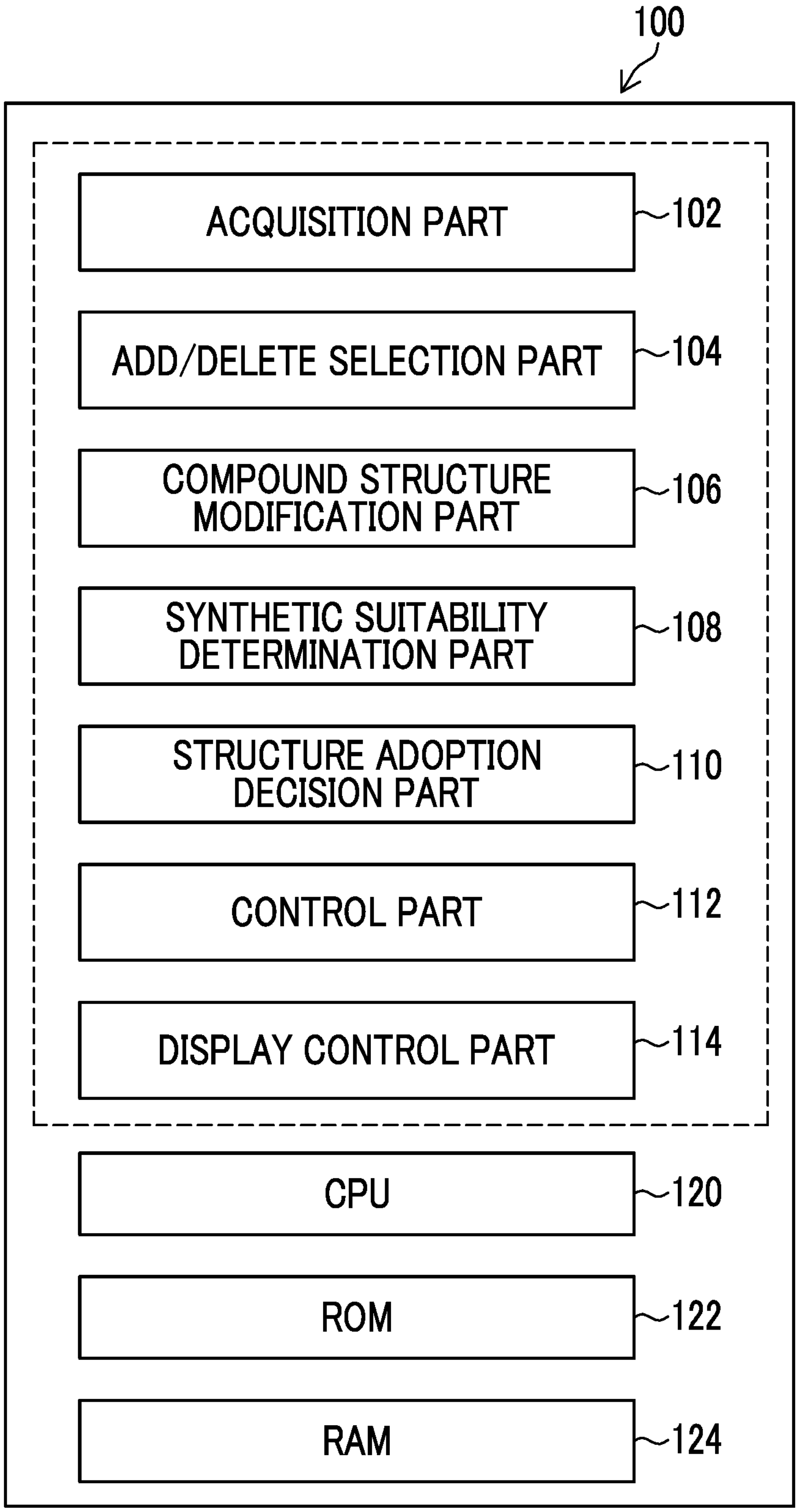
100
ACQUISITION PART
102
ADD/DELETE SELECTION PART
104
COMPOUND STRUCTURE MODIFICATION PART
106
SYNTHETIC SUITABILITY DETERMINATION PART
108
STRUCTURE ADOPTION DECISION PART
110
CONTROL PART
112
DISPLAY CONTROL PART
114
CPU
120
ROM
122
RAM
124

FIG. 3

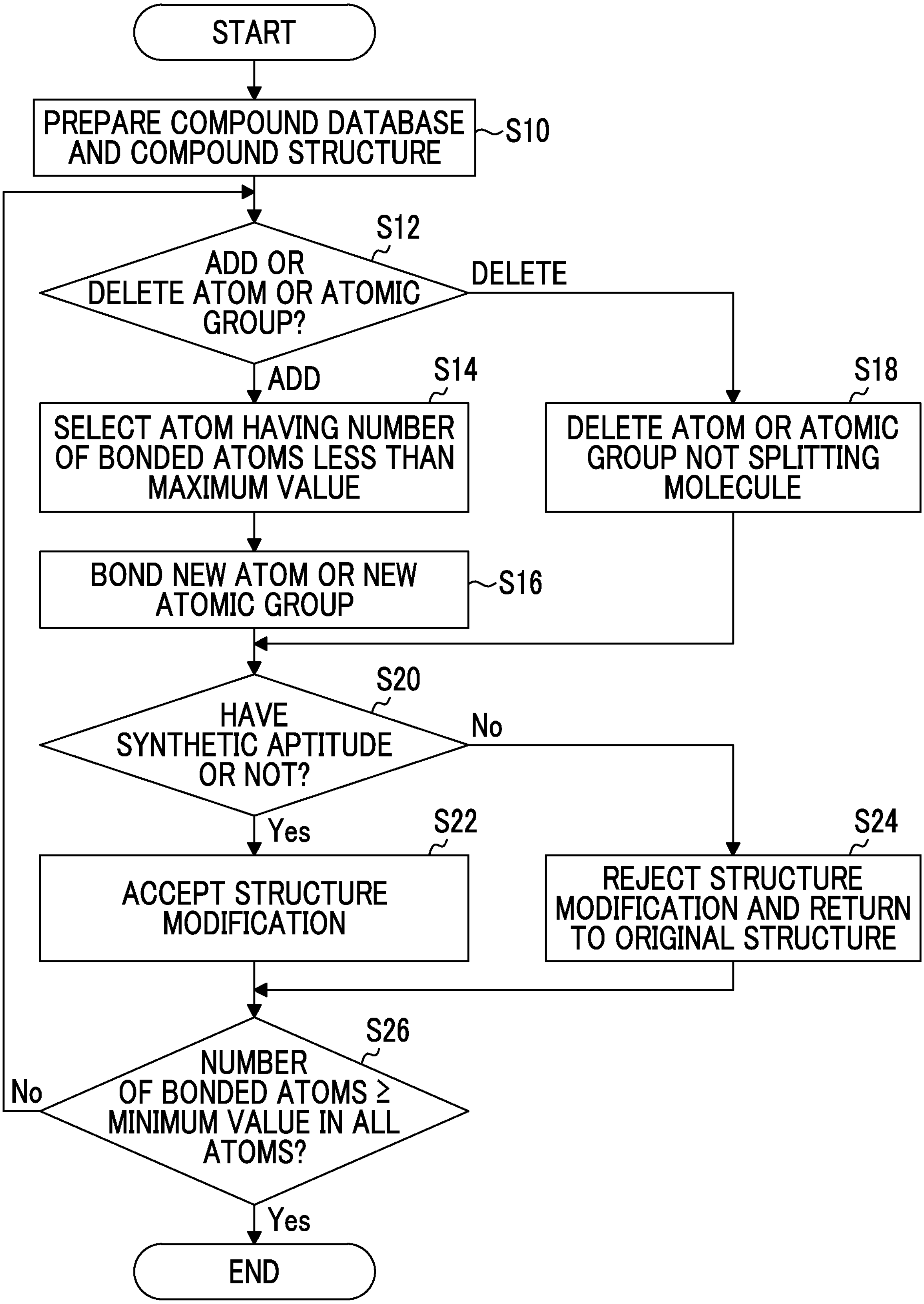
START
PREPARE COMPOUND DATABASE AND COMPOUND STRUCTURE
S10
S12
ADD OR DELETE ATOM OR ATOMIC GROUP?
DELETE
ADD
S14
SELECT ATOM HAVING NUMBER OF BONDED ATOMS LESS THAN MAXIMUM VALUE
S18
DELETE ATOM OR ATOMIC GROUP NOT SPLITTING MOLECULE
BOND NEW ATOM OR NEW ATOMIC GROUP
S16
S20
HAVE SYNTHETIC APTITUDE OR NOT?
No
Yes
S22
ACCEPT STRUCTURE MODIFICATION
S24
REJECT STRUCTURE MODIFICATION AND RETURN TO ORIGINAL STRUCTURE
S26
NUMBER OF BONDED ATOMS ≥ MINIMUM VALUE IN ALL ATOMS?
No
Yes
END

FIG. 4

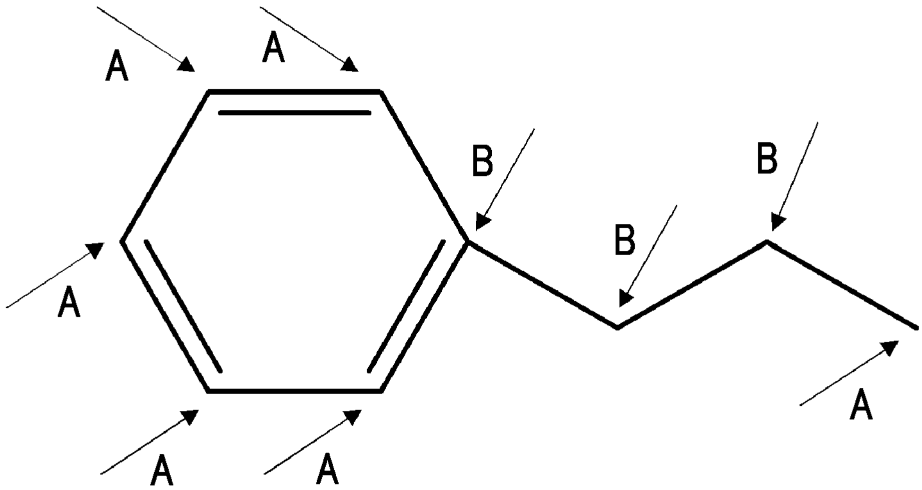

FIG. 5

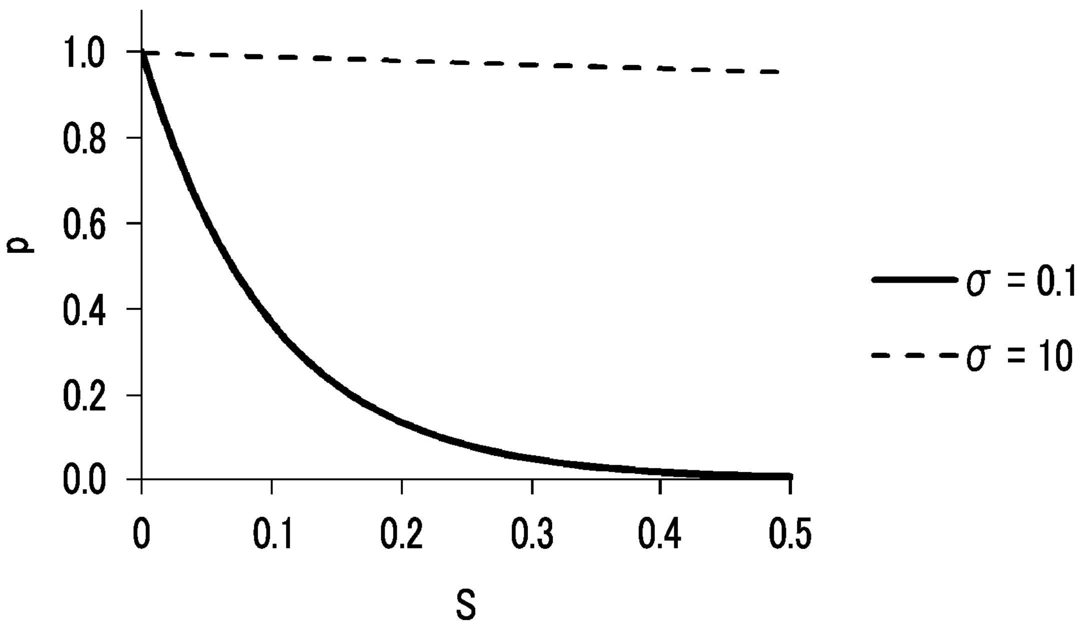

FIG. 6

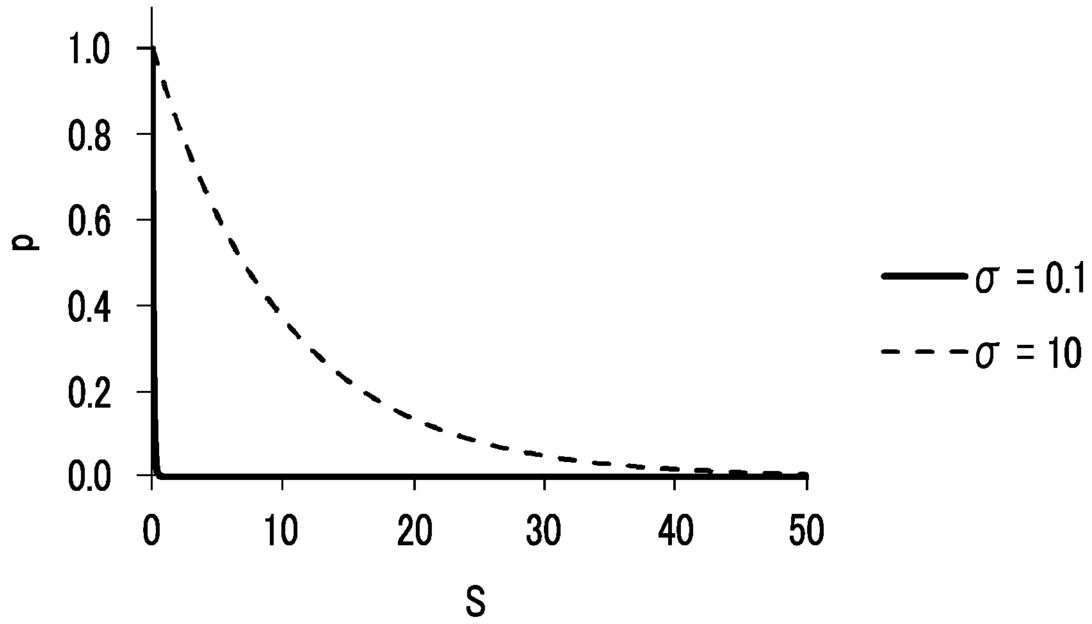
1.0
0.8
0.6
0.4
0.2
0.0
p
0
10
20
30
40
50
S
σ = 0.1
σ = 10

FIG. 7

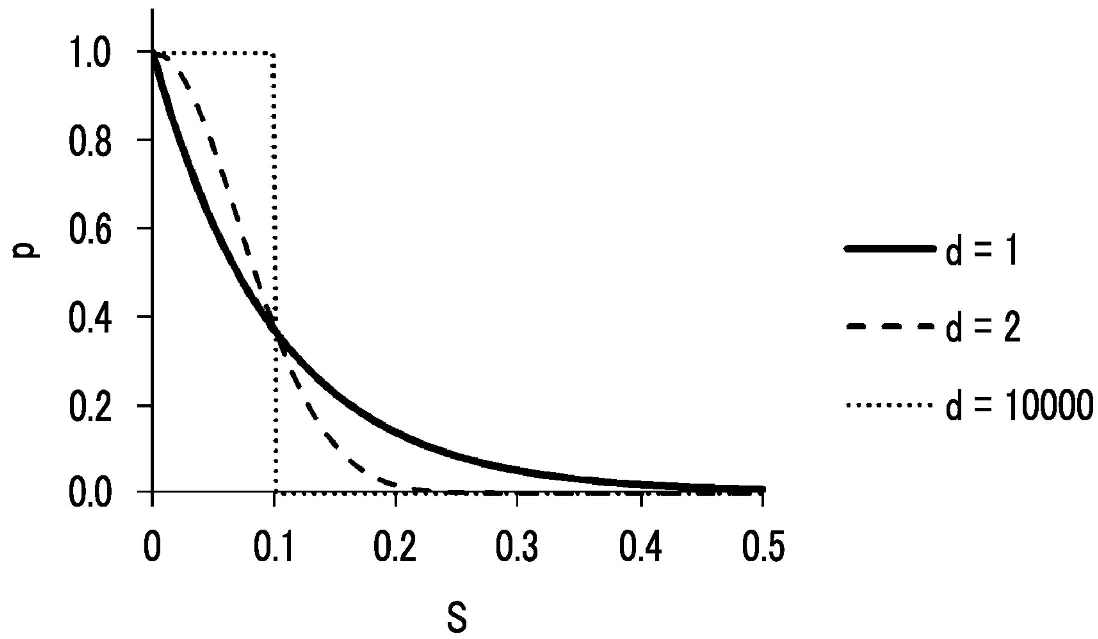
1.0
0.8
0.6
0.4
0.2
0.0
p
0
0.1
0.2
0.3
0.4
0.5
S
d = 1
d = 2
d = 10000

FIG. 8

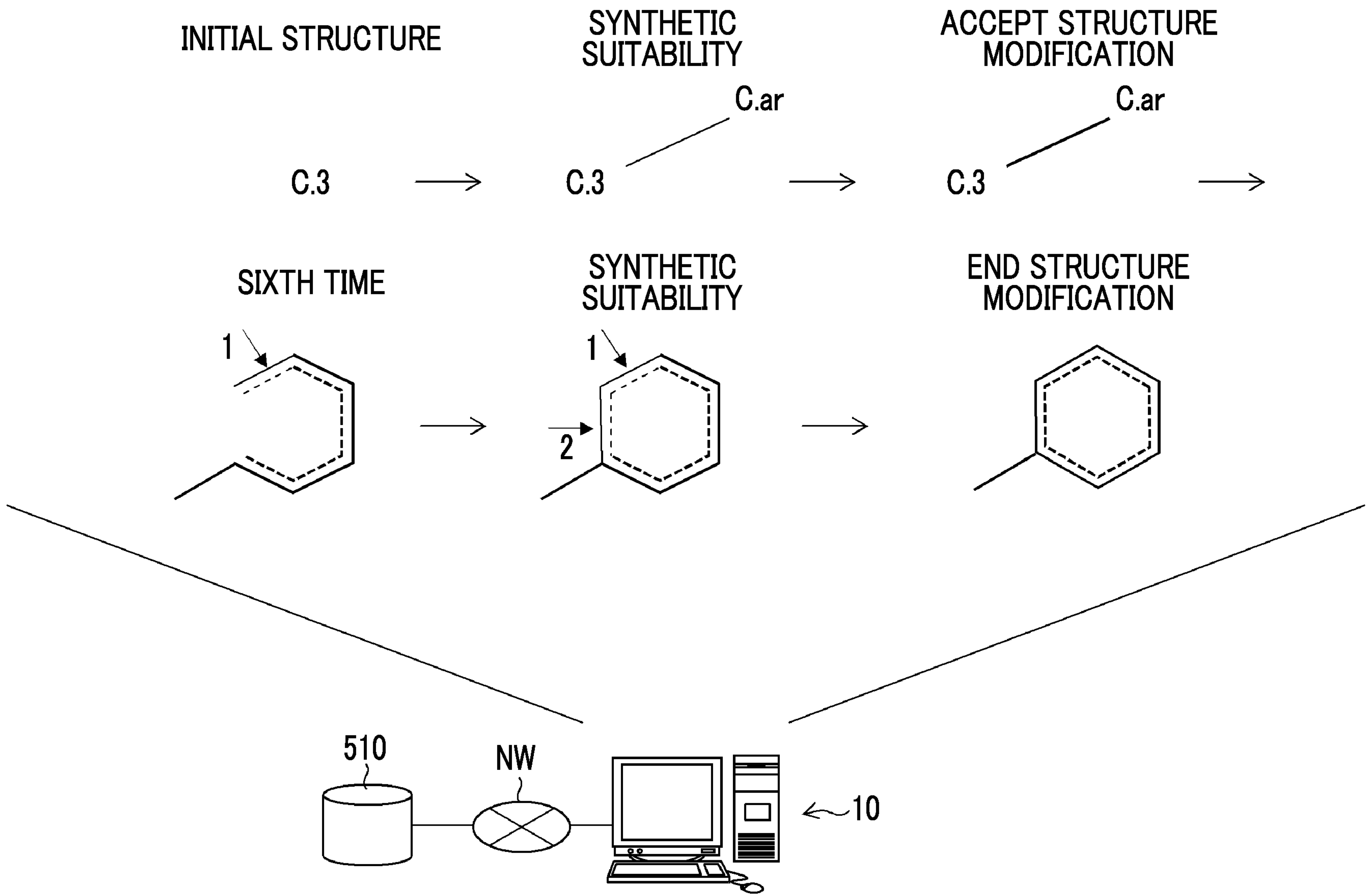
INITIAL STRUCTURE
SYNTHETIC SUITABILITY
ACCEPT STRUCTURE MODIFICATION
SIXTH TIME
SYNTHETIC SUITABILITY
END STRUCTURE MODIFICATION
510
NW
10

FIG. 9

INITIAL STRUCTURE

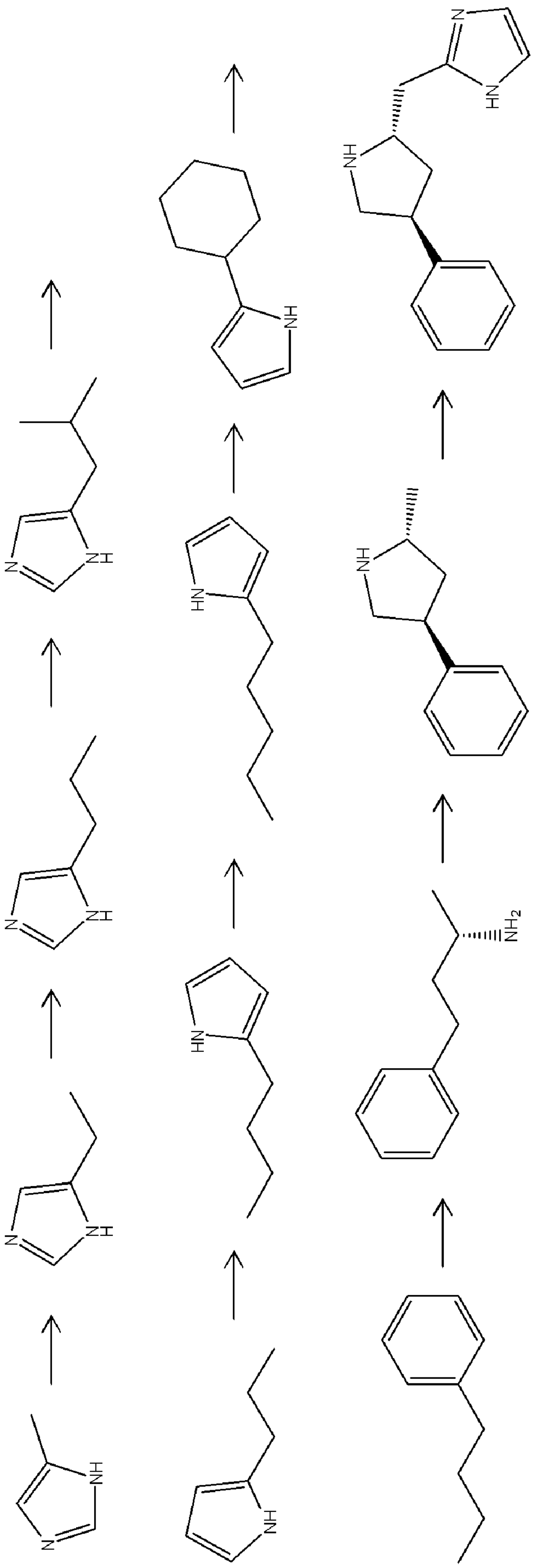

COMPOUND STRUCTURE AFTER MODIFICATION

FIG. 10

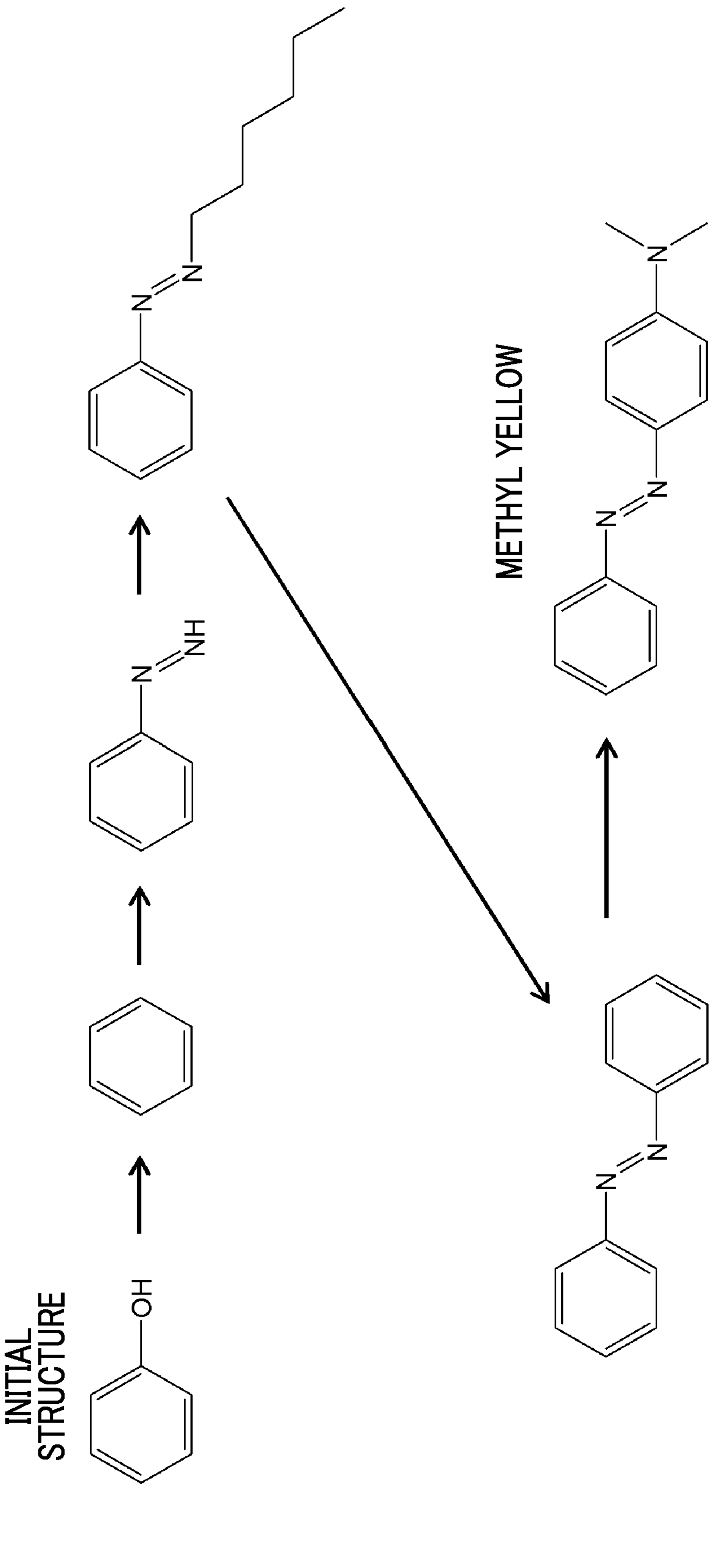

# INTERNATIONAL SEARCH REPORT

International application No.
PCT/JP2019/036073

A. CLASSIFICATION OF SUBJECT MATTER
Int.Cl. G16C20/64(2019.01)i, G16C20/50(2019.01)i

According to International Patent Classification (IPC) or to both national classification and IPC

B. FIELDS SEARCHED
Minimum documentation searched (classification system followed by classification symbols)
Int.Cl. G16C20/64, G16C20/50

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | 河合 健太郎, 構造設計/構造創出のための分子進化アルゴリズム, Journal of Computer Chemistry, Japan, vol. 10, no. 1, 日本コンピュータ化学会, 2011, pp. 25-31, (KAWAI, Kentaro, "Molecular Evolutionary Algorithm for Structure Design and Structure Generation", Society of Computer Chemistry, Japan) | 14<br>1-13 |
| Y | JP 2003-206246 A (YAMATO, Ichiro) 22 July 2003, paragraph [0030] (Family: none) | 1-13 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 03 December 2019 (03.12.2019) | 10 December 2019 (10.12.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No.
PCT/JP2019/036073

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2009/064015 A1 (IN-SILICO SCIENCES, INC.) 22 May 2009, p. 39, line 17 to p. 40, line 4 & US 2010/0312538 A1, paragraph [0189] & EP 2216429 A1 & CN 101855392 A | 1-13 |
| Y | ERTLE, Peter, "Estimation of synthetic accessibility score of drug- like molecules based on molecular complexity and fragment contributions", Journal of Chemiformatics, 10 June 2009, no. 8, pp. 1-11 | 11 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **HISAKI IKEBATA et al.** *Bayesian molecular design with a chemical language,* 23 July 2018, https://www.ncbi.nlm.nih.gov/pubmed/28281211
- RecGen (Refined Compound Generator. Kyoto Constella Technologies Co., Ltd, 23 July 2018
- RecGen (Refined Compound Generator). Kyoto Constella Technologies Co., Ltd, 23 July 2018